# EUROPÄISCHE PATENTANMELDUNG

(11) **EP 2 053 055 A1**
(43) Veröffentlichungstag der Anmeldung: **29.04.2009**
(21) Anmeldenummer: 07075924.6
(22) Anmeldetag: 24.10.2007
(51) Int. Cl.: C07J 1/00

(54) **11.beta.-Fluoro-3-acetoxyestra-3,5-dien-17-on und Verfahren zu seiner Herstellung**

(71) Anmelder: Bayer Schering Pharma Aktiengesellschaft, 13353 Berlin (DE)
(72) Erfinder: Petrov, De, Dr. Orlin, 14159 Berlin (DE); Schneider, De, Dr. Matthias, 10589 Berlin (DE); Bohlmann, De, Dr. Rolf, 14055 Berlin (DE); Vettel, De, Dr. Stephan, 13467 Berlin (DE)

(57) **Zusammenfassung**

Die vorliegende Erfindung betrifft 11β-Fluor-3-acetoxyestra-3,5-dien-17-on als geeignete Zwischenstufe bei der Herstellung von 11-Fluor-substituierten Steroiden und das Verfahren zu dessen Herstellung. Dazu wird 11α-Hydroxyestr-4-en-3,17-dion in einem organischen nicht-protischen Lösungsmittel mit 1 bis 3 Äquivalenten n-Nonafluorbutansulfonylfluorid und 3 bis 5 Äquivalenten Diazabicycloundecen (DBU) bei -40 bis -20 °C umgesetzt und nach einer wässrigen Aufarbeitung mit 5 bis 10 Äquivalenten Essigsäureanhydrid und 0,01 bis 1 Äquivalenten einer starken Säure versetzt. Das gewünschte Produkt fällt spontan aus der Reaktionslösung aus und wird durch Filtration in einer sehr hohen Reinheit gewonnen. Das Verfahren zeichnet sich durch die sehr hohe Ausbeute, Vermeidung einer chromatographischen Aufreinigung des Produktes, einem verringerten Anteil der Abfälle und deutlich erhöhtem Prozessdurchsatz aus. Das erfindungsgemäße Verfahren eignet sich daher insbesondere zur Herstellung von 11β-Fluor-3-acetoxyestra-3,5-dien-17-on in großem technischen Maßstab.

## Beschreibung

Die vorliegende Erfindung betrifft 11β-Fluor-3-acetoxyestra-3,5-dien-17-on (I). 11β-Fluor-3-acetoxyestra-3,5-dien-17-on (I) 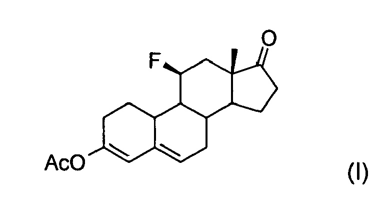 ist als Zwischenstufe für die Herstellung von 11-Fluor-substituierten Steroiden, die als pharmazeutische Wirkstoffe Verwendung finden, geeignet. Des weiteren betrifft die Erfindung ein Verfahren zu dessen Herstellung.

11β-Fluor-7α-substituierte Steroide sind pharmakologisch hochpotente Verbindungen mit einer ausgeprägten androgenen oder antiestrogenen Wirkung. Als Beispiele können die in der WO 2004/011663 und der WO 2002/059139 beschriebenen Androgene, und die in der WO 03/045972, der WO 99/33855 und der WO 98/07740 beschriebenen Antiestrogene angeführt werden.

Ein Schlüsselschritt bei der Synthese dieser Verbindungen ist die Einführung des 11β-FluorSubstituenten durch eine Deoxyfluorierungsreaktion des 11α-Hydroxy-substituierten Vorläufers (WO 2002/059139). Eine etablierte Methode zur direkten Umwandlung von primären und sekundären Alkoholen einschließlich entsprechender Hydroxysteroide in die entsprechenden Fluoride (Deoxyfluorierung) ist in der Literatur beschrieben (z.B. H. Vorbrüggen et al., Tetrahedron Letters, 1995, 2611; J. Yin et al, Organic Letters 2004, 1465; Ch. Marson et al. Synthetic Communications 2002, 2125; US 5,760,255, US 6,248,889). Dabei wird der entsprechende Alkohol mit kommerziell erhältlichem n-Nonafluorbutansulfonylfluorid und einer starken organischen Base (bevorzugt Diazabicycloundecen (DBU)) in einem geeigneten organischen Lösungsmittel (z. B. Toluol, Xylol, Diglyme, Dichlormethan, Hexan, etc.) umgesetzt und nach wässrigem Aufarbeiten, Extraktion und Chromatographie das entsprechende Fluor-Derivat isoliert.

US 6,248,889 beschreibt einen Deoxyfluorierungsprozess, bei dem die Verwendung eines geringen bzw. keines Überschusses an Base als vorteilhaft beschrieben wird.

Vorbrüggen et al. (Tetrahedron Letters, 1995, 2611) beschreiben speziell die Überführung von 11α-Hydroxy-19-norandrost-4-en-3,17-dion (A) in die entsprechende 11β-Fluor-Verbindung durch Umsetzung mit 1.5 Äquivalenten n-Nonafluorbutansulfonylfluorid und 3 Äquivalenten DBU in Toluol bei 24 - 30 °C. Nach einer chromatographischen Aufreinigung erhält man 11β-Fluor-19-norandrost-4-en-3,17-dion (B; Beispiel 1 b), c) in WO 2002/059139) in einer Ausbeute von 66 % d. Th..

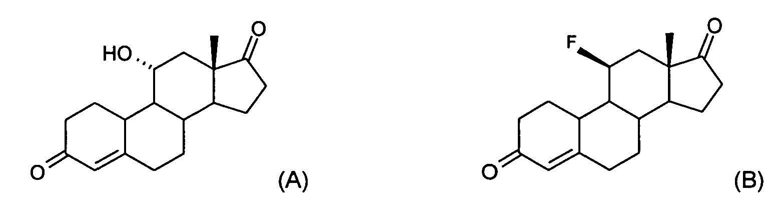

Eine ähnliche Umsetzung (74% Ausbeute nach Chromatographie) mit 1.5 Äquivalenten n-Nonafluorbutansulfonylfluorid und 2.8 Äquivalenten DBU bei 0 °C ist in DE 10104327 beschrieben.

Nachteil dieser Methoden ist neben der mässigen Ausbeute der schlechte Prozessdurchsatz, da große Mengen Lösungsmittel sowohl bei der Reaktion, als auch bei der mehrfachen Extraktion während der Aufarbeitung, erforderlich sind. In der Regel erhält man nach der Reaktionsaufarbeitung ein Rohprodukt, dessen Menge 3 mal größer ist als die Menge des eingesetzten Ausgangsmaterials. Zur Isolierung des Produktes aus dem Rohgemisch ist eine chromatographische Reinigung unumgänglich. Daher sind solche Verfahren zur Herstellung von 11β-Fluorsteroiden in Multikilogramm-Mengen nicht geeignet.

Aufgabe der vorliegenden Erfindung ist es daher, ein Verfahren zur Herstellung von 11β-Fluor-3-acetoxyestra-3,5-dien-17-on (I) zu schaffen, welches eine einfache Gewinnung des Produktes gestattet und sich durch eine gute Ausbeute und einen guten Prozessdurchsatz auszeichnet.

Erfindungsgemäß wurde nunmehr gefunden, dass nach der temperaturkontrollierten Reaktion von 11β-Hydroxy-estr-4-en-3,17-dion (A) mit 1,5 - 2 Äquivalenten n-Nonafluorbutansulfonylfluorid in Gegenwart eines Überschusses von mindestens 3,3 Äquivalenten DBU bei - 40 bis - 20°C, gefolgt von einer pH-kontrollierten wässrigen Aufarbeitung und anschliessender Acetylierung des Zwischenproduktes das entstandene 11β-Fluor-3-acetoxyestra-3,5-dien-17-on überraschenderweise spontan aus dem Reaktionsgemisch kristallisiert und in einer sehr hohen Ausbeute (79 - 86% d.Th.) durch einfache Filtration des Reaktionsgemisches gewonnen werden kann.

Als Lösungsmittel für die Deoxyfluorierungsreaktion können aprotische Lösungsmittel wie zum Beispiel Methylenchlorid, Toluol, Ethylacetat, Isopropylacetat oder Benzotrifluorid dienen. Bevorzugt wird Ethylacetat angewendet.

Für den zweiten Prozessschritt (Acetylierungsreaktion) werden Essigsäureanhydrid oder Isopropenylacetat, bzw. Vinylacetat in Gegenwart starker Säuren wie zum Beispiel p-Toluolsulfonsäure (p-TsOH), Methansulfonsäure, Schwefelsäure oder Bromwasserstoff (HBr) verwendet.

Bevorzugt wird Essigsäureanhydrid in Anwesenheit einer katalytischen Menge p-TsOH verwendet.

Das so in guter Ausbeute und Reinheit gewonnene 11 β-Fluor-3-acetoxyestra-3,5-dien-17-on kann durch Hydrolyse in einem, dem Fachmann bekannten Bromierungs-/Dehydrobromierungs-Prozess in das entsprechende 11β-Fluor-19-norandrost-4,6-dien-3,17-dion (WO 2002/059139; Beispiel 1 c) überführt werden.

Dieses wiederum kann nach bekannten Verfahren zu Androgenen oder Antiestrogenen weiter umgesetzt werden.

Die nachfolgenden Beispiele dienen der näheren Erläuterung der Erfindung. Alle Temperaturen sind in Grad Celsius (unkorrigiert) und alle Mengenangaben sind in Gewichtsprozent angegeben, soweit nicht anders vermerkt.

### Beispiele:

### 11-β-Fluor-3-acetoxyestra-3,5-dien-17-on

**1.** Eine Mischung aus 50 g 11α-Hydroxyestre-4-en-3,17-dion und 85,6 ml DBU (3,3 Äqu.) in 250 ml Ethylacetat wird auf -35°C bis -40°C abgekühlt. Dazu wird eine Lösung von 50 ml n-Nonafluorbutansulfonylfluorid (1,6 Äqu.) in 100 ml Ethylacetat bei -35°C unter starkem Rühren langsam zugetropft. Die Reaktionslösung wird 15 Stunden bei -35°C gerührt, bis laut HPLC der Anteil 11α-Hydroxyestr-4-en-3,17-dion < 1 % beträgt. Danach wird auf -10°C erwärmt, 60 min. nachgerührt und die Reaktion mit 20 ml Wasser versetzt.
   Die Kühlung wird entfernt und die Reaktionsmischung mit 100 ml 2N Schwefelsäure versetzt. Es wird 90 min bei 10°C nachgerührt. Die Phasen werden getrennt und die organische Phase wird mit 22 ml 2N Schwefelsäure versetzt, um einen pH-Wert von 2 einzustellen. Die Phasen werden wieder getrennt und die organische Phase wird mit 50 ml ges. NaHCO₃-Lösung und mit 50 ml ges. Kochsalzlösung gewaschen und unter vermindertem Druck auf ca. 160 ml eingeengt. Es wird zweimal mit jeweils 200 ml Ethylacetat versetzt und bei 60°C und vermindertem Druck auf ca. 160 ml eingeengt. Es entsteht jeweils ein rührbarer Kristallbrei.
   Zu dem rührbaren Kristallbrei werden bei 20°C 147,1 ml Essigsäureanhydrid (9 Äqu.) zugegeben. Es wird auf 0°C gekühlt. Innerhalb von 4 h werden in 3 Portionen insgesamt 2,24 ml Methansulfonsäure (0,4 Äqu.) zugegeben Es wird weitere 44 Stunden bei 0°C gerührt und anschliessend das ausgefallene Reaktionsprodukt abgesaugt und viermal mit 40 ml eiskaltem Isopropylacetat gewaschen und bei 40°C im Vakuum getrocknet.
   Man erhält 49,6 g (86,1% d. Th.) 11-β-Fluor-3-acetoxyestra-3,5-dien-17-on als hellbeigen, kristallinen Feststoff. Smp. 175-177 °C; [α]_{D} = - 32.3° in CHCl₃; H-NMR (δ in CHCl₃): 5.82 [1 H, d, *J* = 2 Hz ], 5.50 [1 H, m], 5.08 [1 H, d(br), *J* = 49 Hz], 2.15 [3 H, s], 1.04 [3 H, d, *J* = 1.5 Hz] [ppm].
**2.** 0,5 kg 11α-Hydroxyestr-4-en-3,17-dion und 856 ml DBU (3,3 Äqu.) werden in 3,25 I Essigester suspendiert und auf -35°C bis -40°C abgekühlt. Dazu wird innerhalb von 120 min eine Lösung von 500 ml n-Nonafluorbutansulfonylfluorid (1,6 Äqu.) in 250 ml Ethylacetat zugegeben.

Die Reaktionslösung wird nach beendeter Zugabe 6 Stunden bei -35°C gerührt Danach wird innerhalb von 30 min auf -10°C erwärmt. Man lässt noch 120 min bei -10°C nachrühren und versetzt die Reaktion mit 600 ml 2N Schwefelsäure. Es wird 60 min nachgerührt und dabei auf 30°C erwärmt.

Die Phasen werden getrennt und die organische Phase wird mit 600 ml 2N Schwefelsäure versetzt, um einen pH 3 einzustellen. Die Phasen werden wieder getrennt und die organische Phase wird mit 800 ml ges. Natriumhydrogencarbonatlösung gewaschen und bei 60°C und 120 mbar auf ca. 1,8 I eingeengt. Es wird zweimal mit jeweils 1,5 I Ethylacetat versetzt und bei 60°C und 120 mbar auf ca. 1,8 I eingeengt. Es entsteht jeweils ein rührbarer Kristallbrei der anschliessend mit 1,471 l Essigsäureanhydrid (9 Äqu.) versetzt wird. Das Gemisch wird auf 0°C gekühlt und nach einer Zugabe von 132 g p-Toluolsulfonsäure (0,4 Äqu.) 24 Stunden bei 0°C gerührt. Das ausgefallene Reaktionsprodukt wird abgesaugt, fünfmal mit 500 ml eiskaltem Ethylacetat gewaschen und bei 40°C im Vakuum getrocknet.

Man erhält 475 g (82,5% d. Th.) 11-β-Fluor-3-acetoxyestra-3,5-dien-17-on als hellbeigen, kristallinen Feststoff.

### Hydrolyse von 11β-Fluor-3-acetoxyestra-3,5-dien-17-on zu 11β-Fluor-19-norandrost-4-en-3,17-dion

950 g 11 β-Fluor-3-acetoxyestra-3,5-dien-17-on werden in 10 Methanol gelöst. Dazu werden 2,0 I einer gesättigten Kaliumcarbonat-Lösung bei 50°C zugegeben. Nach 5 Stunden wird das Reaktionsgemisch auf Raumtemperatur abgekühlt und auf ca. 3 I im Vakuum aufkonzentriert. Das ausgefallene Produkt wird abfiltriert, mit Wasser gewaschen und getrocknet. Man erhält 755 g (91% d.Th.) 11β-Fluor-19-norandrost-4-en-3,17-dion als weissen Feststoff. Schmp. 171-173°C.

## Patentansprüche

1. 11 β-Fluor-3-acetoxyestra-3,5-dien-17-on.

2. Verfahren zur Herstellung von 11β-Fluor-3-acetoxyestra-3,5-dien-17-on, **dadurch gekennzeichnet, dass** 11α-Hydroxyestr-4-en-3,17-dion mit 1 bis 3 Äquivalenten n-Nonafluorbutansulfonylfluorid und 3 bis 5 Äquivalenten Diazabicycloundecen (DBU) bei -40 bis -20 °C in einem organischen nicht-protischen Lösungsmittel reagieren gelassen und nach einer wässrigen Zwischenaufarbeitung mit 5 bis 10 Äquivalenten eines Acetylierungsmittels in Gegenwart von 0,01 bis 1 Äquivalenten einer starken Säure umgesetzt und das ausgefallene Produkt durch Filtration des Reaktionsgemisches gewonnen wird.

3. Verfahren zur Herstellung von 11β-Fluor-3-acetoxyestra-3,5-dien-17-on nach Anspruch 2, **dadurch gekennzeichnet, dass** mehr als 3 Äquivalente DBU verwendet werden.

4. Verfahren zur Herstellung von 11β-Fluor-3-acetoxyestra-3,5-dien-17-on nach Anspruch 2, **dadurch gekennzeichnet, dass** Ethylacetat als Lösungsmittel verwendet wird.

5. Verfahren zur Herstellung von 11β-Fluor-3-acetoxyestra-3,5-dien-17-on nach Anspruch 2, **dadurch gekennzeichnet, dass** mit Essigsäureanhydrid als Acetylierungsmittel umgesetzt wird.

6. Verfahren zur Herstellung von 11β-Fluor-3-acetoxyestra-3,5-dien-17-on nach Anspruch 2, **dadurch gekennzeichnet, dass** mit p-Toluolsulfonsäure (p-TsOH) als starker Säure umgesetzt wird.
